# EUROPEAN PATENT SPECIFICATION

(11) **EP 4 403 129 B1**
(45) Date of publication and mention of the grant of the patent: **25.11.2026**
(21) Application number: 24181283.3
(22) Date of filing: 07.12.2016
(51) Int. Cl.: A61B 17/12, A61B 90/00

(54) **OCCLUSION DEVICE**
OKKLUSIONSVORRICHTUNG
DISPOSITIF D'OCCLUSION

(30) Priority: 07.12.2015 US 201562264171 P
(43) Date of publication of application: 24.07.2024
(62) Document number(s) of the earlier application(s) in accordance with Art. 76 EPC: 22154998.3 / 4 011 303; 16808631.2 / 3 386 402
(73) Proprietor: Stryker Ireland Technology Ltd., Cork T45 HX08 (IE)
(72) Inventor: GRIFFIN, Stephen, San Jose (US)
(74) Representative: Schott, Jakob Valentin

(56) References cited:
- WO-A1-2012/034135
- WO-A2-2011/057002
- US-A1- 2001 041 900
- US-A1- 2002 169 473

## Description

### FIELD OF THE INVENTION

The occlusion device disclosed herein relates generally to the field of occlusion devices and/or occlusion device systems and/or implantable occlusion devices and the use of the same for the occlusion of vessels and/or the treatment and/or amelioration of aneurysms and/or for peripheral vascular embolization (a process well known in the art and known to involve the shutdown of blood flow distal to a specified vascular point), for example, in the treatment and/or amelioration of peripheral arterial or venous pathologies and/or any related pathologies requiring vessel occlusion for the treatment thereof.

### BACKGROUND OF THE DISCLOSURE

There is a significant demand for the development of improved occlusion devices and/or systems for the treatment and/or amelioration of aneurysms. This observation is supported by the abundance and wide-range of current occlusion devices and/or systems currently in the aneurysm peripheral vascular embolization treatment field. However, there still remains an unmet need for providing aneurysm treatment and/or amelioration, particularly for neurovascular aneurysms, via occlusion devices comprised of a deployable material designed to achieve greater flow disruption and compartmentalization to introduce stasis and/or designed in such a manner so as to occlude larger and/or more irregularly shaped aneurysms.

It is well known that an aneurysm forms when a dilated portion of an artery is stretched thin from the pressure of the blood. The weakened part of the artery forms a bulge, or a ballooning area, that risks leak and/or rupture. When a neurovascular aneurysm ruptures, it causes bleeding into the compartment surrounding the brain, the subarachnoid space, causing a subarachnoid hemorrhage. Subarachnoid hemorrhage from a ruptured neurovascular aneurysm can lead to a hemorrhagic stroke, brain damage, and death. Approximately 25 percent of all patients with a neurovascular aneurysm suffer a subarachnoid hemorrhage. Neurovascular aneurysms occur in two to five percent of the population and more commonly in women than men. It is estimated that as many as 18 million people currently living in the United States will develop a neurovascular aneurysm during their lifetime. Annually, the incidence of subarachnoid hemorrhage in the United States exceeds 30,000 people. Ten to fifteen percent of these patients die before reaching the hospital and over 50 percent die within the first thirty days after rupture. Of those who survive, about half suffer some permanent neurological deficit.

Smoking, hypertension, traumatic head injury, alcohol abuse, use of hormonal contraception, family history of brain aneurysms, and other inherited disorders such as Ehlers-Danlos syndrome (EDS), polycystic kidney disease, and Marfan syndrome possibly contribute to neurovascular aneurysms.

Most unruptured aneurysms are asymptomatic. Some people with unruptured aneurysms experience some or all of the following symptoms: peripheral vision deficits, thinking or processing problems, speech complications, perceptual problems, sudden changes in behavior, loss of balance and coordination, decreased concentration, short term memory difficulty, and fatigue. Symptoms of a ruptured neurovascular aneurysm include nausea and vomiting, stiff neck or neck pain, blurred or double vision, pain above and behind the eye, dilated pupils, sensitivity to light, and loss of sensation. Sometimes patients describing "the worst headache of my life" are experiencing one of the symptoms of a ruptured neurovascular aneurysm.

Most aneurysms remain undetected until a rupture occurs. Aneurysms, however, may be discovered during routine medical exams or diagnostic procedures for other health problems. Diagnosis of a ruptured cerebral aneurysm is commonly made by finding signs of subarachnoid hemorrhage on a CT scan (Computerized Tomography). If the CT scan is negative but a ruptured aneurysm is still suspected, a lumbar puncture is performed to detect blood in the cerebrospinal fluid (CSF) that surrounds the brain and spinal cord.

To determine the exact location, size, and shape of an aneurysm, neuroradiologists use either cerebral angiography or tomographic angiography. Cerebral angiography, the traditional method, involves introducing a catheter into an artery (usually in the leg) and steering it through the blood vessels of the body to the artery involved by the aneurysm. A special dye, called a contrast agent, is injected into the patient's artery and its distribution is shown on X-ray projections. This method may not detect some aneurysms due to overlapping structures or spasm.

Computed Tomographic Angiography (CTA) is an alternative to the traditional method and can be performed without the need for arterial catheterization. This test combines a regular CT scan with a contrast dye injected into a vein. Once the dye is injected into a vein, it travels to the brain arteries, and images are created using a CT scan. These images show exactly how blood flows into the brain arteries. New diagnostic modalities promise to supplement both classical and conventional diagnostic studies with less-invasive imaging and possibly provide more accurate 3-dimensional anatomic information relative to aneurismal pathology. Better imaging, combined with the development of improved minimally invasive treatments, will enable physicians to increasingly detect, and treat, more silent aneurysms before problems arise.

Several methods of treating aneurysms have been attempted, with varying degrees of success. For example, open craniotomy is a procedure by which an aneurysm is located, and treated, extravascularly. This type of procedure has significant disadvantages. For example, the patient undergoes a great deal of trauma in the area of the aneurysm by virtue of the fact that the surgeon must sever various tissues in order to reach the aneurysm. In treating cerebral aneurysms extravascularly, for instance, the surgeon must typically remove a portion of the patient's skull, and must also traumatize brain tissue in order to reach the aneurysm. As such, there is a potential for the development of epilepsy in the patients due to the surgery.

Other techniques used in treating aneurysms are performed endovascularly. Such techniques typically involve attempting to form a mass within the sac of the aneurysm. Typically, a microcatheter is used to access the aneurysm. The distal tip of the microcatheter is placed within the sac of the aneurysm, and the microcatheter is used to inject embolic material into the sac of the aneurysm. The embolic material includes, for example, detachable coils or an embolic agent, such as a liquid polymer. The injection of these types of embolic materials suffers from disadvantages, most of which are associated with migration of the embolic material out of the aneurysm into the parent artery. This can cause permanent and irreversible occlusion of the parent artery.

For example, when detachable coils are used to occlude an aneurysm which does not have a well-defined neck region, the detachable coils can migrate out of the sac of the aneurysm and into the parent artery. Further, it is at times difficult to gauge exactly how full the sac of the aneurysm is when detachable coils are deployed. Therefore, there is a risk of overfilling the aneurysm in which case the detachable coils also spill out into the parent artery.

Another disadvantage of detachable coils involves coil compaction over time. After filling the aneurysm, there remains space between the coils. Continued hemodynamic forces from the circulation act to compact the coil mass resulting in a cavity in the aneurysm neck. Thus, the aneurysm can recanalize.

Embolic agent migration is also a problem. For instance, where a liquid polymer is injected into the sac of the aneurysm, it can migrate out of the sac of the aneurysm due to the hemodynamics of the system. This can also lead to irreversible occlusion of the parent vessel.

Techniques have been attempted in order to deal with the disadvantages associated with embolic material migration to the parent vessel. Such techniques are, without limitation, temporary flow arrest and parent vessel occlusion, and typically involve temporarily occluding the parent vessel proximal of the aneurysm, so that no blood flow occurs through the parent vessel, until a thrombotic mass has formed in the sac of the aneurysm. In theory, this helps reduce the tendency of the embolic material to migrate out of the aneurysm sac. However, it has been found that a thrombotic mass can dissolve through normal lysis of blood. Also, in certain cases, it is highly undesirable from a patient's risk/benefit perspective to occlude the parent vessel, even temporarily. Therefore, this technique is, at times, not available as a treatment option. In addition, it is now known that even occluding the parent vessel may not prevent all embolic material migration into the parent vessel.

Another endovascular technique for treating aneurysms involves inserting a detachable balloon into the sac of the aneurysm using a microcatheter. The detachable balloon is then inflated using saline and/or contrast fluid. The balloon is then detached from the microcatheter and left within the sac of the aneurysm in an attempt to fill the sac of the aneurysm. However, detachable balloons also suffer disadvantages and as such this practice has all but been superseded by the current practice of deployment of coils or other types of occlusion devices. For example, detachable balloons, when inflated, typically will not conform to the interior configuration of the aneurysm sac. Instead, the detachable balloon requires the aneurysm sac to conform to the exterior surface of the detachable balloon. Thus, there is an increased risk that the detachable balloon will rupture the sac of the aneurysm. Further, detachable balloons can rupture and migrate out of the aneurysm.

Another endovascular technique for treating aneurysms involves occlusion devices having two expandable lobes and a waist, or an expandable body portion, a neck portion, and a base portion.

Still another endovascular technique for treating aneurysms involves occlusion devices for intrasaccular implantation having a body portion designed to fill and/or expand radially into the space within the sac of the aneurysm.

Still another endovascular technique is disclosed in the co-owned pending application, U. S. Serial Number 14/699,188.

Many current occlusion devices are not designed for treatment of large aneurysms or for aneurysms of irregular shapes and sizes, including wide-and narrow-necked aneurysms, side-wall and bifurcation aneurysms, for example. Many current occlusion devices are constructed of braided or woven mesh designs and such designs, if reconfigured for a large and irregular shaped aneurysm, would typically utilize too much material. This would make it difficult to collapse down into a constrained, low profile, delivery configuration small enough to be delivered and deployed without excess friction on the walls of the delivery catheter or other delivery lumen. The sheer bulkiness of these devices would make them inconvenient or inappropriate for intra-cranial delivery.

Therefore, the occlusion device disclosed herein provides innovative improvements and several advantages in the field of vascular occlusion devices because the occlusion device disclosed herein provides aneurysm and/or body lumen treatment and/or amelioration, particularly for neurovascular aneurysms of large and irregular sizes, via the use of super compactable continuous mesh-based fully-retrievable deployable material. The occlusion devices disclosed herein are comprised of a mesh-based deployable continuous structure having compressible axial mesh carriages configured end to end and defined on either end by pinch points in the continuous mesh structure. This novel design achieves greater flow disruption and compartmentalization within the aneurysm or body lumen and results in increased stasis particularly so as to occlude larger and more irregularly shaped aneurysms.

WO 2012/034135 A1 discloses devices and methods for treating vascular defects, such as, for example, balloon-type aneurysms. In one embodiment, an apparatus includes an insertion portion and an expandable implant. The expandable implant is configured to be deployed in an aneurysm and is coupled to the insertion portion. The expandable implant has a first portion and a second portion coupled to the first portion. The expandable implant is movable between a first configuration in which the first portion and the second portion are substantially linearly aligned and a second configuration in which the second portion at least partially overlaps the first portion.

### SUMMARY OF THE INVENTION

The present inventor has designed an intra-aneurysmal occlusion device for deploying into the aneurysm sac providing aneurysm treatment and/or amelioration through the creation of flow disruption and ultimate stasis. The occlusion device uniquely comprises a series of compressible mesh carriages which have discreet pinch points defining the carriages at each end and providing a continuous 3-dimentional mesh network inside the aneurysm for flow disruption, thrombus establishment, and/or a framework for cell growth. Such an implantable occlusion device is also used for treatment of vessel occlusion and/or peripheral vascular embolization.

Disclosed herein is an occlusion device for implantation into a body lumen or aneurysm, the occlusion device comprising a continuous compressible mesh structure comprising axial mesh carriages configured end to end, wherein each end of each carriage is a pinch point in the continuous mesh structure.

In one embodiment, a marker encircles at least one pinch point of the continuous mesh structure. In a further embodiment, the marker is radiopaque.

In another embodiment, at least one mesh carriage of the continuous mesh structure comprises an inner coaxial mesh carriage or inner coaxial mesh carriages. In a further embodiment, the inner coaxial mesh carriage or carriages is dissimilar material to its outer mesh carriage. In a further embodiment, the inner coaxial mesh carriages are two (2) or three (3) inner coaxial mesh carriages. In another further embodiment, the inner coaxial mesh carriage or carriages is dissimilar mesh density to its outer mesh carriage.

In another embodiment, the continuous mesh structure expands to a deployed shape and fills the body lumen or aneurysm.

In another embodiment, the number (n) of axial mesh carriages is two (2), three (3), four (4) or five (5) axial mesh carriages.

Also disclosed herein is a kit for treatment and/or amelioration of a body lumen or an aneurysm; the kit comprising an occlusion device for implantation into a body lumen or aneurysm comprising, a continuous compressible mesh structure comprising axial mesh carriages configured end to end, wherein each end of each carriage is a pinch point in the continuous mesh structure; and a delivery system or detachment system corresponding to the occlusion device. In one embodiment, the delivery system of the kit is a microcatheter, catheter, guide wire, or pusher wire. In another embodiment, the detachment system of the kit is an electrolytic detachment system.

Also disclosed herein is a method for treating or ameliorating a body lumen or an aneurysm in a patient, the method comprising delivering to a body lumen or an aneurysm an occlusion device comprising, a continuous compressible mesh structure comprising axial mesh carriages configured end to end, wherein each end of each carriage is a pinch point in the continuous mesh structure; and deploying the occlusion device in the aneurysm, thereby treating or ameliorating the aneurysm in the patient.

In another embodiment, disclosed herein is an occlusion device for implantation into a body lumen or aneurysm comprising, a compressible continuous mesh structure comprising an axial mesh carriage, wherein each end of the carriage is a pinch point in the continuous mesh structure, and wherein the carriage comprises inner coaxial mesh carriages.

Additionally disclosed herein are methods for manufacture and/or delivery and/or deployment of the occlusion device disclosed herein.

In other embodiments, the occlusion device in the preceding paragraphs may incorporate any of the preceding or subsequently disclosed embodiments.

The Summary of the Invention is not intended to define the claims nor is it intended to limit the scope of the invention in any manner.

Other features and advantages of the invention will be apparent from the following Drawings, Detailed Description, and the Claims.

### BRIEF DESCRIPTION OF THE FIGURES

Figure 1A-1B illustrates perspective views of an embodiment of an occlusion device disclosed herein. Figure 1A shows a mesh carriage embodiment of the occlusion device disclosed herein. Figure 1B shows a series of mesh carriages of the occlusion device disclosed herein.
Figure 2 illustrates a perspective view of an embodiment of a series of variable mesh carriages of the occlusion device disclosed herein.
Figure 3 illustrates perspective views of an embodiment of the delivery and/or deployment of an occlusion device disclosed herein having a series of mesh carriages.
Figure 4 illustrates perspective views of an embodiment of an occlusion device disclosed herein having a series of mesh carriages deployed in an aneurysm.
Figure 5 illustrates a perspective view of an embodiment of an occlusion device disclosed herein having a mesh carriage comprising an inner coaxial mesh carriage.
Figure 6 illustrates a perspective view of an embodiment of an occlusion device disclosed herein having a mesh carriage comprising inner coaxial mesh carriages.
Figure 7 illustrates a perspective view of an embodiment of the electrolytic delivery and/or deployment and/or detachment of an occlusion device disclosed herein and shows the delivery via a catheter lumen and/or pusher wire having electrolytic means of an occlusion device disclosed herein having mesh carriages and inner mesh carriages.
Figure 8 illustrates a perspective view of an embodiment of the electrolytic delivery and/or deployment and/or detachment of an occlusion device disclosed herein and shows device deployment and electrolytic detachment of a pusher wire from the occlusion device disclosed herein having a series of mesh carriages and inner coaxial mesh carriages.
Figure 9 illustrates a perspective view of an embodiment of an occlusion device disclosed herein having a series of two variably sized mesh carriages.
Figure 10 illustrates a perspective view of an embodiment of an occlusion device disclosed herein having a series of two variably sized mesh carriages deployed in an aneurysm.

### DETAILED DESCRIPTION

The occlusion device disclosed herein is illustrated in the drawings and description in which like elements are assigned the same reference numerals. However, while particular embodiments are illustrated in the drawings, there is no intention to limit the occlusion device disclosed herein to the specific embodiment or embodiments disclosed. Rather, the occlusion device disclosed herein is intended to cover all modifications, alternative constructions, and equivalents falling within the scope of the invention. As such, the drawings are intended to be illustrative and not restrictive.

Unless otherwise defined, all technical terms used herein have the same meaning as commonly understood by one of ordinary skill in the art to which this technology belongs.

In this description, the non-SI unit "inch" is used. 1 inch equals to 2,54 cm.

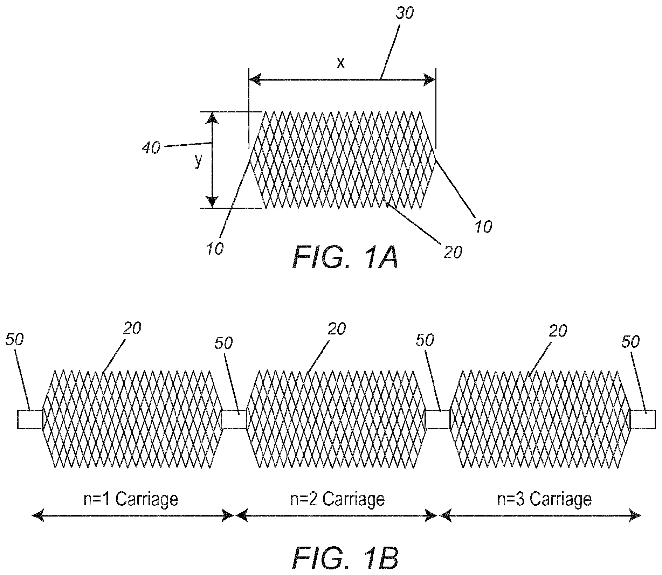

Exemplary embodiments of the occlusion device disclosed herein are depicted in Figures 1-10.

For the purposes of the occlusion device disclosed herein, the terminology "corresponds to" means there is a functional and/or mechanical relationship between objects which correspond to each other. For example, an occlusion device delivery system corresponds to (or is compatible with) an occlusion device for deployment thereof.

For the purposes of the occlusion device disclosed herein, the terminology "occlusion device" means and/or may be interchangeable with terminology such as, without limitation, "device" or "occlusion device system" or "occlusion system" or "system" or "occlusion device implant" or "implant" or "intrasaccular implant" or "intra-aneurysmal implant" and the like.

Occlusion device delivery systems are well known and readily available in the art. For example, such delivery technologies may be found, without limitation, in US Patent and Publication Numbers 4,991,602; 5,067,489; 6,833,003; 2006/0167494; and 2007/0288083; each of the teachings of which are incorporated herein. For the purposes of the occlusion device disclosed herein, any type of occlusion device delivery means and/or delivery system and/or delivery technology and/or delivery mechanism and/or detachment (and/or attachment) means and/or detachment system and/or detachment technology and/or detachment mechanism may be utilized and/or modified in such a manner as to make compatible (so as to correspond) with the occlusion device disclosed herein. Exemplary occlusion device delivery mechanisms and/or systems include, without limitation, guide wires, pusher wires, catheters, micro-catheters, and the like. Exemplary occlusion device detachment mechanisms include, without limitation, fluid pressure, electrolytic mechanisms, hydraulic mechanisms, interlocking mechanisms, and the like. In one embodiment, the occlusion device disclosed herein is used in a method of electrolytic detachment. Electrolytic detachment is well known in the art and can be found, for example, in US Patent Numbers 5,122,136; 5,423,829; 5,624,449; 5,891,128; 6,123,714; 6,589,230; and 6,620,152.

The occlusion device disclosed herein comprises a series of compressible mesh **20** carriages having discreet **10** pinch points at each end of each **20** carriage. In this respect, the occlusion device design is one continuous 3-dimensional mesh network which, when deployed in an **90** aneurysm or body lumen, provides flow disruption, thrombus establishment, a framework for cell growth, and/or ultimate stasis. For the purposes of the claimed invention, a **20** "carriage" is an axial segment of mesh between each **10** pinch point or each **50** marker-encircled **10** pinch point. A **10** "pinch point" is located at and defines the ends of an axial segment of mesh. Such segmented mesh **20** carriages and **10** pinch points are, in many instances, within a continuous mesh structure or network. A "pinch point" is as a constrained and gathered location on the mesh structure which functions to restrict movement of the adjacent carriages at an isolated point and thereby stabilizes the carriages relative to each other. The number (n) of these **20** carriages is as many as clinically and practically possible, and determined by a clinician in accordance with known diagnostic techniques in advance, for treating large and/or irregular-sized **90** aneurysms, and for delivery through about a 150 centimeter (cm) **70** catheter (or micro-catheter). The **30** length (x) of each **20** carriage can vary depending on the number (n) of **20** carriages deemed appropriate to occlude an **90** aneurysm of a given size so long as the **30** length (x) is sufficient to permit the **20** carriage to expand to dimension y (40 width) in "free air." As is accepted in the art, the diameter of such an occlusion device is measured in free air. The **40** width (y) of each **20** carriage ranges (in free air) from about 2 millimeters (mm) to about 50 mm in order to be clinically practical. When deployed, the **20** carriage compresses in such a manner where the diameter or **40** width (y) grows or expands up to about a factor of two (2) such that a **20** carriage of **40** y dimension is capable of growing to approximately 2 times **40** y (or 2y). In other words, each **20** carriage compresses like a marshmallow which causes **30** x to be reduced and **40** y to expand. In one embodiment, in free air, each **20** carriage can be designed in such a manner that **30** x is greater or equal to **40** y but in the deployed (compressed) shape, **40** y is greater than **30** x. Such an occlusion device comprising a series of compressible **20** carriages can be constructed in a variable manner so as to select the number (n) of the **20** carriages as well as the **30** length (x) and **40** width (y) of each **20** carriage to accommodate a wide range of sizes and shapes of **90** aneurysms or body lumen to be treated. As such, in another embodiment, in free air, each **20** carriage can be designed in such a manner that **30** x is equal to or less than **40** y and in the deployed (compressed) shape, **40** y remains greater than **30** x.

In one embodiment, the device is constructed of a metal braid of readily available material such as, without limitation, nitinol (NiTi), cobalt chrome (CoCr) alloy, stainless steel, tungsten iridium alloy or a combination thereof. For example, the mesh **20** carriages are woven with the most clinically relevant and practical braided mesh in a range of as few as 36 braids to as many as 144 braids. In another embodiment, the angle of the weave of the metal braid construction creates the softest compressible mesh design. For example, the mesh is braided with a wire diameter of about 0.0075 inches up to about .005 inches. Accordingly, the occlusion device disclosed herein is a series of ultra-soft axially compressible mesh **20** carriages, wherein n = to the number of mesh **20** carriages in a single occlusion device. Prior to use of such an occlusion device having "n" carriages, a clinician or physician determines the size and shape of the aneurysm or body lumen to be treated using readily available diagnostic techniques. The physician or clinician is then able to best choose the occlusion device having the desired number (n) of carriages, and various sizes thereof, which will be used to best treat the given aneurysm or body lumen.

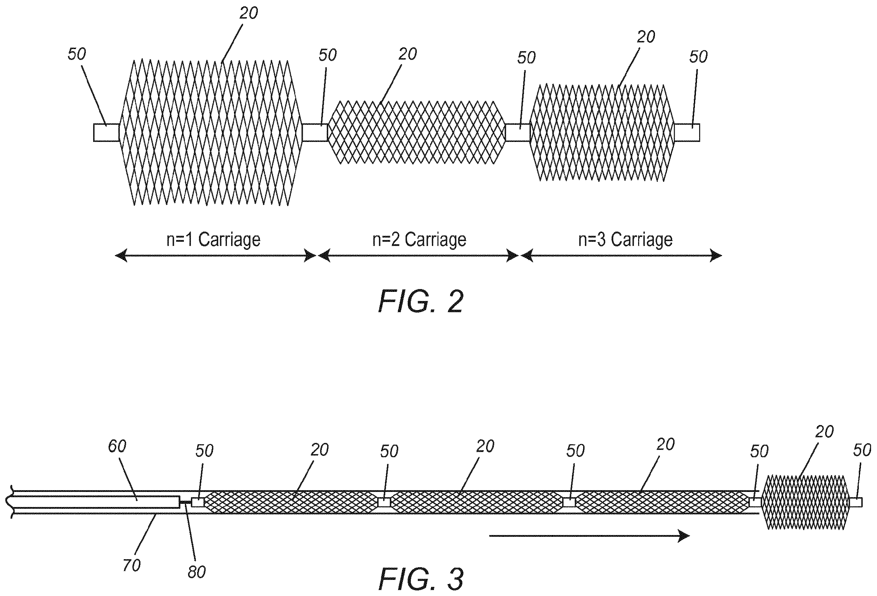

Figure 1A shows an exemplary embodiment of a singular mesh **20** carriage having an axial **30** length (x) which is greater than its **40** width (y) in free air, and wherein each end of the **20** carriage is a **10** pinch point. In another embodiment, the axial **20** carriage can have an axial **30** length (x) which is equal to its **40** width (y) in free air. In yet another embodiment, the axial **20** carriage can have an axial **30** length (x) which is less than its **40** width (y) in free air. Figure 1B shows an embodiment of a series of axial mesh segmented **20** carriages in a continuous compressible mesh structure, wherein n = three (3) axial mesh **20** carriages, and wherein each **10** pinch point is encircled by a **50** marker. "Markers" are well known and readily available in the medical device art. In some embodiments, a marker consists of metallic material, often radiopaque material, and takes the form of a shape such as a band-shaped marker, a ring-shaped marker, a tube-shaped, and the like, so as to encircle a pinch point of the occlusion device. Alternatively, a marker may consist of wire strands wound around and therefore encircling a given pinch point. In one embodiment, the **50** markers which encircle each **10** pinch point provide positional reference under X-Ray as to where the device is located in the **70** catheter (or microcatheter) and where the device is located once deployed in an **90** aneurysm or body lumen. Figure 2 shows an exemplary embodiment of a series of variably-sized (differing in size and/or diameter) segmented axial mesh **20** carriages in a continuous compressible mesh structure, wherein n = three (3) axial mesh **20** carriages, wherein each **20** carriage varies with respect to the dimensions of **30** x and **40** y, and wherein each **10** pinch point is encircled by a **50** marker. In such a configuration as shown in Figure 2, the first **20** carriage (on the right) to enter the **90** aneurysm will have a diameter or **40** width (y) which is slightly larger than the largest measured diameter of an **90** aneurysm to be treated which ensures the continuous mesh structure of the device will not come in contact with the dome of the **90** aneurysm which often is the thinnest and most sensitive area of the **90** aneurysm's wall. Additionally, in this configuration as shown in Figure 2, the diameter or **40** width (y) of the last **20** carriage (on the left) to enter the **90** aneurysm will be larger than the **100** aneurysm neck which ensures this last **20** carriage remains secure (and is anchored) inside the **90** aneurysm.

Figure 3 shows an embodiment of the occlusion device as disclosed herein with the constrained mesh **20** carriages of the device loaded within a **70** catheter lumen. The device is advanced with an electrolytic **60** pusher wire having an **80** electrolytic detachment zone and when deployed, the **20** carriage expands as it exits the **70** catheter tip to occupy the volume of the **90** aneurysm or body lumen. As the subsequent axial **20** carriages are deployed into the **90** aneurysm, they compress against each other filling the **90** aneurysm thereby creating a series of layers and/or compartments inside the **90** aneurysm or body lumen which disrupts flow and ultimately creates stasis.

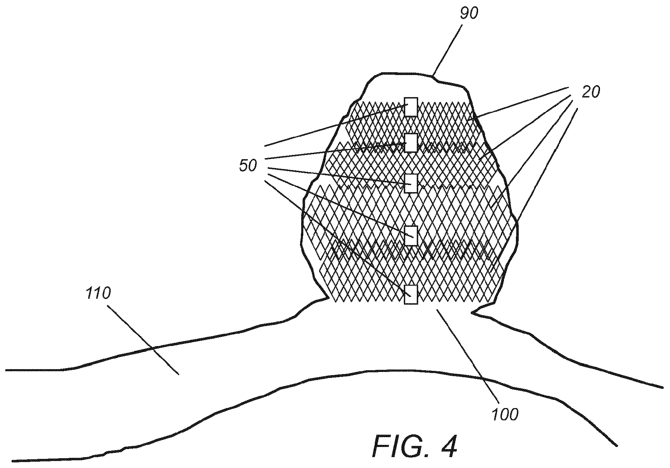

Figure 4 shows an embodiment of an occlusion device as disclosed herein deployed within an **90** aneurysm to be treated. The compressible mesh **20** carriages create multiple layers of compartmentalization inside the **90** aneurysm sac. In this deployed shape, the axial **20** carriages fill the **90** aneurysm or body lumen as layers or compartments thereby transforming the deployed axial **30** length (x) to a proportion of its axial **30** length in free air. For example, deployed axial **30** length (x) is about 5% to about 50% of the axial **30** length in free air.

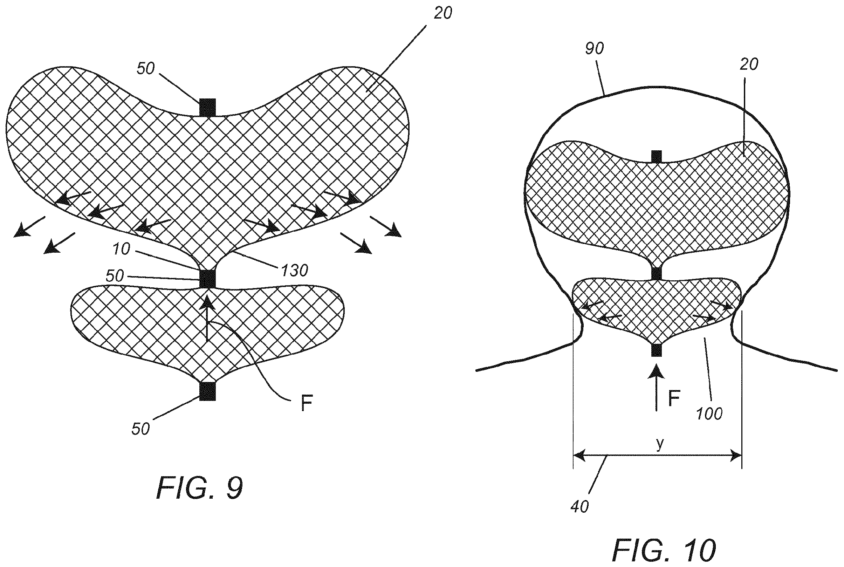

Figures 9 and 10 show embodiments of an occlusion device as disclosed herein. In such embodiments, the distal **20** carriage (top **20** carriage in Figure 9 & 10) expands into the **90** aneurysm and creates a stable structure for the variably sized, smaller subsequent **20** carriage to push against thereby stabilizing the device as it sits across (or within) the **100** aneurysm neck. In such a configuration, and in one embodiment, the hourglass-like **130** shape of the distal **20** carriage leading into the **10** pinch point and/or **50** marker located between the **20** carriages contributes to overall stability of the device. In another embodiment, the hourglass-like **130** shape of the distal **20** carriage is defined by its projection outward toward the subsequent **20** carriage rather than recessing into itself. Figure 9 and 10 show the distribution of forces with arrows. Force (F) distributes forces or pressure into the distal **20** carriage which embeds and secures the device stably into place within the **90** aneurysm. Like other variable configurations of the series of **20** carriages shown in Figure 2, Figure 10 shows that the diameter or **40** width (y) of the last **20** carriage to enter the **90** aneurysm will be larger than the **100** aneurysm neck which ensures this last **20** carriage remains secure (and is anchored) inside the **90** aneurysm so as to disrupt blood flow and ultimately create stasis.

Without wishing to be bound by theory, this configuration of a continuous compressible mesh structure divided into segments of axial **20** carriages triggers a mechanism of action believed to contribute to enhanced acute thrombogenicity of the device in animal studies. It is also believed that the localizing of a small volume of clot between the series of **20** carriage layers and compartments, which have a high surface area contribution from the wire strands, facilitates nucleating and stabilizing thrombus in an **90** aneurysm. This compartmentalization of the occlusion device in its deployed shape is an effective stabilizing or anchoring feature of the deployed device as blood applies pressure to the mesh structure distributed across or within the **100** neck of the aneurysm. Such a configuration also provides sufficient apposition of the compressible device against the **90** aneurysm wall or vessel wall for peripheral arterial or venous occlusion. The device disclosed herein provides sufficient mesh density to confer stasis acutely and the wire mesh/braid distribution remains relatively uniform in deployment.

In another embodiment of an occlusion device disclosed herein, the compressible axial mesh **20** carriages comprise a relatively uniform distribution of wire mesh strands or braids such as, without limitation, a 72 NiTi wire mesh strand braided configuration or a combination of 72 NiTi and CoCr wire mesh strand braided configuration. In other embodiments, the occlusion device comprises wire mesh strands or braids that range from 36 to 144 NiTi strand braided configuration.

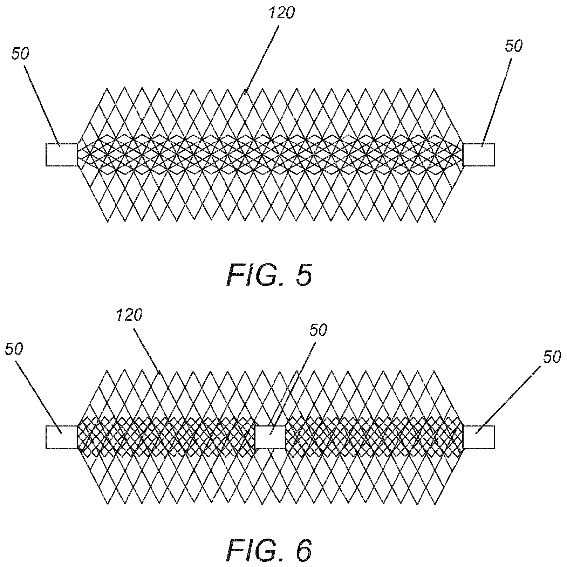

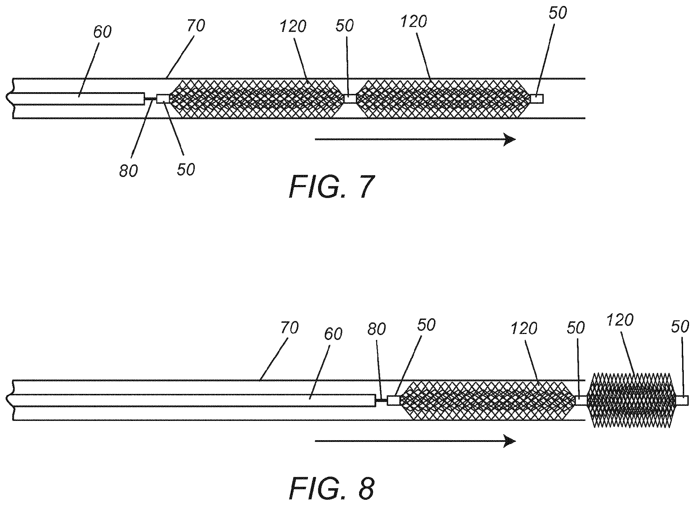

Figure 5 shows a singular compressible axial mesh **20** carriage comprising a **120** coaxial inner mesh carriage. Figure 6 shows a singular axial mesh **20** carriage comprising **120** coaxial inner mesh carriages. Such a **120** coaxial mesh inner or **120** coaxial mesh inner creates greater flow disruption and compartmentalization than an axial mesh **20** carriage without a **120** coaxial mesh inner carriage, thereby introducing stasis and thrombus stabilization. In another embodiment, the axial **20** carriage and the **120** coaxial carriage (or carriages) are constructed of dissimilar metal mesh. In a further embodiment, the dissimilar metal mesh creates a galvanic effect which can further enhance thrombus development. In another further embodiment, the dissimilar metal mesh can be comprised of one metal in one **20** carriage which possesses radiopaque properties relative to the metal in the other 20, **120** carriage and thus enhances visualization of the device. In such embodiments, braid mesh density can be the same or different in axial outer **20** carriages and **120** coaxial inner carriages and wires of the inner and outer mesh can have different numbers of strands and wire diameters. Such a **120** coaxial carriage or coaxial carriages are variable in dimension compared to the outer axial **20** carriage. For example, in one embodiment, a **120** coaxial carriage or carriages can range from about 5% to about 95% of the dimensions of the outer axial **20** carriage of which the **120** coaxial carriage or coaxial carriages is/are comprised within. Figure 7 shows the **20** axial and **120** coaxial carriages loaded in the **70** catheter lumen and Figure 8 shows electrolytic device deployment with an electrolytic detachable **60** pusher wire and the expansion of the 20, **120** carriages (outer and inner) exiting the **70** catheter tip.

In one embodiment, a **50** marker such as a ring encircles the **10** pinch points defining each end of each 20, **120** carriage of the continuous mesh structure. As such, the **50** marker of the occlusion device disclosed herein is a substantially solid collar or rigid member such as, without limitation a solid ring or band comprised of materials such as, without limitation, gold, platinum, stainless steel, and/or combinations thereof. In another embodiment, radiopaque materials such as, without limitation, gold, platinum, platinum/iridium alloy, and/or combinations thereof, can be used. Such a **50** marker provides positional visualization of the device during delivery and placement. The **50** markers are located on the occlusion device encircling **10** pinch points on each end of each 20, **120** carriage. In this manner, the **50** marker located at the proximal end of the proximal 20, **120** carriage is capable of resting above or within the **100** neck of an **90** aneurysm. The solidness of the **50** markers help confer stability of the device within the **90** aneurysm and prevents movement or the transfer of forces through the compressible mesh **20, 120** carriages thereby preventing misplacement or accidental movement of the device. The **50** markers are also configured with a junction to cooperate and release from/attach to a corresponding delivery means such as, without limitation, a delivery **70** catheter or **60** guide wire and/or pusher wire technologies. It also advantageously provides for full retrievability of the device disclosed herein.

In another embodiment, the substantially solid **50** marker comprises a radiopaque material (such as for example, without limitation, platinum, gold, platinum/iridium alloy, and/or combinations thereof) to facilitate visualization of the occlusion device under fluoroscopy during delivery, placement and/or deployment. The **50** marker comprises a proximal end and a distal end. Occlusion devices disclosed herein may be configured to incorporate the use of markers to influence shape, diameter, and/or curvature of the compressible 20, **120** carriages upon expansion during deployment. Additionally, the **50** marker may be designed in various shapes to influence the overall profile of the occlusion device having a series of mesh 20, **120** carriages to ensure a proper fit of the expanded/deployed occlusion device within the **90** aneurysm sac.

Figures 3, 7 and 8 show exemplary means for electrolytic delivery and/or deployment and/or detachment of the occlusion device disclosed herein through an artery and/or **110** vessel adjacent to the **90** aneurysm or body lumen. Electrolytic detachment means and methods such as U.S. Patent 5,122,136 are well known in the art. In one embodiment, a coil-wound **60** core wire (or guide wire or pusher wire) of the **70** catheter (or micro-catheter) is attached inside the **50** marker at its most distal end to the occlusion device disclosed herein (as shown in Figures 3, 7 and 8). The coil wind maintains a constant diameter (ϕ) so as not to impact upon flexibility or stiffness of the delivery **70** catheter or micro-catheter or **60** guide wire. In certain embodiments, FEP (Fluorinated Ethylene Propylene) heat shrink tubing encases the coil-wound portion of the core wire. Numerous readily available and well known attachment techniques in the medical device arts can be used to attach the distal end of the core wire inside the marker and to the occlusion device or implant. Such attachment techniques include, without limitation, adhesives, laser melting, laser tack, spot, and/or continuous welding. In one embodiment, an adhesive is used to attach the distal end of the core wire inside the marker. In a further embodiment, the adhesive is an epoxy material which is cured or hardened through the application of heat or UV (ultra-violet) radiation. In an even further embodiment, the epoxy is a thermal cured, two-part epoxy such as EPO-TEK^{®} 353ND-4 available from Epoxy Technology, Inc., 14 Fortune Drive, Billerica, Mass. Such an adhesive or epoxy material encapsulates the junction of the core wire inside the **50** marker and increases its mechanical stability.

In another embodiment, during and/or after deployment of the device, the coil-wound **60** core wire detaches the occlusion device disclosed herein at an **80** electrolytic detachment site (or zone) on the **60** core wire itself in such a manner so that the **60** core wire is severed and/or dissolved through electrolytic action at the base of the **50** marker. Such action then releases and/or places the occlusion device into an **90** aneurysm or vessel to be treated.

In certain embodiments, the compressible mesh structure of the occlusion device disclosed herein can be filled with an embolic material to promote clotting and closure of the **90** aneurysm.

In other embodiments, the occlusion device disclosed herein may further incorporate adjunctive elements and/or members such as coiling techniques, framing coils, embolic agents, additional markers, polymers, resorbent polymers and/or a combination thereof.

Resilient and compressible mesh materials for design and/or manufacture of occlusion devices are readily available and well known by those skilled in the relevant art. As such, resilient and compressible mesh materials range from a wide variety of available materials such as, without limitation, nickel titanium (nitinol or otherwise known as NiTi), stainless steel, polymers, and/or combinations thereof. Exemplary known biomedical polymeric families include, without limitation, polymers such as polyphosphazenes, polyanhydrides, polyacetals, poly(ortho esters), polyphosphoesters, polycaprolactones, polyurethanes, polylactides, polycarbonates, polyamides, and/or a combination thereof. (See, e.g., J Polym Sci B Polym Phys. Author manuscript; available in PMC 2012 June 15.)

In one exemplary embodiment, the resilient and compressible mesh material is formed of woven strands of polymer material, such as, without limitation, nylon, polypropylene or polyester. The polymer strands can be filled with a radiopaque material which allows the physician treating the aneurysm to fluoroscopically visualize the location of the device within the vasculature. Radiopaque filler materials preferably include bismuth trioxide, tungsten, titanium dioxide or barium sulfate, or radiopaque dyes such as iodine. The resilient and compressible mesh material can be formed by strands of radiopaque material. The radiopaque strands allow the physician and/or radiologist to fluoroscopically visualize the location of the mesh, without the use of filled polymer materials. Such radiopaque strands may be formed with materials such as, without limitation, gold, platinum, a platinum/iridium alloy, and/or a combination thereof. In one embodiment, the resilient mesh material is constructed of 10%-45% platinum core NiTi. In another embodiment, the resilient mesh material is constructed of 10% platinum core NiTi, 15% platinum core NiTi, 20% platinum core NiTi, or 45% platinum core NiTi. 10% platinum core NiTi construction is sufficient to provide a ghost image of the occlusion device under x-ray.

Such constructed combination wires or composite wires having a radiopaque core and non-radiopaque outer layer or casing are readily available and well known in the medical device and metallic arts as DFT^{®} (drawn-filled-tube) wires, cables or ribbons. DFT^{®} wire is a metal-to-metal composite constructed to combine the desired physical and mechanical attributes of two or more materials into a single wire. By placing the more radiopaque, but more ductile material in the core of the wire, the NiTi outer layer is able to provide the resulting composite wire with similar mechanical properties of a 100% NiTi wire. DFT^{®} wires are available from Fort Wayne Metals Corp., Fort Wayne, Ind., U.S.A. See also, for example, the journal article entitled Biocompatible Wire by Schaffer in Advanced Materials & Processes, Oct 2002, pages 51-54.

Where the compressible mesh structure is formed of radiopaque metal strands, the strands may be covered with a polymer coating or extrusion. The coating or extrusion over the radiopaque wire strands provides fluoroscopic visualization but also increases the resistance of the strands to bending fatigue and may also increase lubricity of the strands. The polymer coating or extrusion, in one embodiment, is coated or treated with an agent which tends to resist clotting, such as heparin. Such clot resistant coatings are generally known. The polymer coating or extrusion can be any suitable extrudable polymer, or any polymer that can be applied in a thin coating, such as Teflon^{®} or polyurethane.

In yet another embodiment, the strands of the compressible mesh structure are formed using both metal and polymer braided strands. Combining the metal strands with the polymer strands into a braid changes the flexibility characteristics of mesh. The force required to deploy and/or collapse such a mesh portion is significantly reduced over that required for a mesh portion that includes only metal mesh strands. However, the radiopaque characteristics of the mesh for fluoroscopic visualization are retained. Metal strands forming such a device includes, without limitation, stainless steel, gold, platinum, platinum/iridium, nitinol, and/or combinations thereof. Polymer strands forming the device can include nylon, polypropylene, polyester, Teflon^{®}, and/or combinations thereof. Further, polymer strands of the mesh material can be chemically modified to make them radiopaque with known techniques such as, without limitation, by using gold deposition onto the polymer strands, or by using ion beam plasma deposition of suitable metal ions onto the polymer strands.

The compressible mesh structure can also be formed with filaments or strands of varying diameter and/or varying flexibility. For example, wire diameters for use in the occlusion device disclosed herein range from about 0.0075 inches up to about .005 inches. By varying the size or flexibility of the polymer strands, the flexibility characteristics of the mesh, upon deployment, can also be varied. By varying the flexibility characteristics, both the deployed (compressed) and delivery (constrained) configuration of the resilient and compressible mesh structure can be varied or changed to substantially any desired shape.

Not only can the mesh be formed of both polymer strands or filaments and metal strands or filaments, but it can be formed using filaments of different polymer materials. For example, different polymer materials having different flexibility characteristics can be used in forming the mesh. This alters the flexibility characteristics to change the resultant configuration of the mesh structure in both the deployed and the collapsed positions. Such biomedical polymers are readily known and available in the art and can be derived from polymeric families such as, without limitation, polyphosphazenes, polyanhydrides, polyacetals, poly (ortho esters), polyphosphoesters, polycaprolactones, polyurethanes, polylactides, polycarbonates, polyamides, and/or a combination thereof.

Compressible mesh materials suitable for use within the mesh carriages may take the form of a flat woven sheet, knitted sheet, or a laser cut wire mesh. In general, the material should include two or more sets of substantially parallel strands, with one set of parallel strands being at a pitch of between 45 degrees and 135 degrees with respect to the other set of parallel strands. In some embodiments, the two sets of parallel strands forming the mesh material are substantially perpendicular to each other. The pitch and general construction of the mesh material may be optimized to meet the performance needs of the occlusion device.

The wire strands of the metal fabric used in the occlusion device disclosed herein should be formed of a material which is both resilient and compressible and can be heat-treated to substantially set a desired shape. Materials which are believed to be suitable for this purpose include a cobalt-based low thermal expansion alloy referred to in the field of occlusion devices as Elgiloy^{®}, nickel-based high-temperature high-strength "superalloys" commercially available from Haynes International under the trade name Hastelloy^{®}, nickel-based heat treatable alloys sold under the name Incoloy^{®} by International Nickel, and a number of different grades of stainless steel. The important factor in choosing a suitable material for the wires is that the wires retain a suitable amount of the deformation induced by the molding surface (or shape memory, as described below) when subjected to a predetermined heat treatment.

One class of materials which meet these qualifications are so-called shape memory alloys. Such alloys tend to have a temperature induced phase change which will cause the material to have a preferred configuration which can be fixed by heating the material above a certain transition temperature to induce a change in the phase of the material. When the alloy is cooled, the alloy will "remember" the shape it was in during the heat treatment and will tend to assume that same and/or similar configuration unless constrained from doing so.

One particular shape memory alloy for use in the occlusion device disclosed herein is nitinol, an approximately stoichiometric alloy of nickel and titanium, which may also include other minor amounts of other metals to achieve desired properties. NiTi alloys such as nitinol, including appropriate compositions and handling requirements, are well known in the art and such alloys need not be discussed in detail here. For example, United States Patent Numbers 5,067,489 and 4,991, 602, discuss the use of shape memory NiTi alloys in guide wire-based technologies. Such NiTi alloys are preferred, at least in part, because they are commercially available and more is known about handling such alloys than other known shape memory alloys. NiTi alloys are also very elastic. Indeed, they are said to be known as "superelastic" or "pseudoelastic." This elasticity will help an occlusion device as disclosed herein return to prior expanded configuration for deployment thereof.

The wire strands can comprise a standard monofilament of the selected material, *i.e.,* a standard wire stock may be used. In some embodiments, 72 wire strands and/or 72 strand braid configuration is used. In other embodiments, the occlusion device comprises wire mesh strands or braids that range from 36 to 144 NiTi strand braided configurations. If so desired, though, the individual wire strands may be formed from "cables" made up of a plurality of individual wires. For example, cables formed of metal wires where several wires are helically wrapped about a central wire are commercially available and NiTi cables having an outer diameter of 0.003 inches or less can be purchased. One advantage of certain cables is that they tend to be "softer" than the monofilament wires having the same diameter and formed of same material. Additionally, the use of a cable can increase the effective surface area of the wire strand, which will tend to promote thrombosis.

An occlusion device disclosed herein is configured with a continuous mesh structure having a series of compressible axial **20** carriages (defined by **10** pinch points) having a mesh density sufficient for functioning in such a manner as an endothelial cell scaffold layers or compartments filling a vessel or body lumen or **90** aneurysm and thereby reducing blood flow by about 60% to trigger clot formation and/or healing of the **90** aneurysm and/or ultimate stasis. For the purposes of the occlusion device disclosed herein, the terminology "mesh density" means the level of porosity or the ratio of metal to open area of the mesh structure. Mesh density relates to the number and size of the openings or pores of the mesh and by the extent that the pores are open or closed in situations where opening or pore openness varies between delivery and deployment. Generally, a high mesh density region of a resilient mesh material has approximately about 40% or more metal area and about 60% or less open area.

In some embodiments, the compressible mesh structure may be formed uniformly of the same material; however such material may have different knitted, stitched, braided, and/or cut construction.

In other embodiments, the implantable occlusion device disclosed herein can be used for the process of peripheral vascular embolization (a process well known in the art and known to involve the shutdown of blood flow distal to a specified vascular point), for example, in the treatment and/or amelioration of peripheral arterial or venous pathologies and/or any related pathologies requiring vessel occlusion for the treatment thereof.

The occlusion device disclosed herein may incorporate reasonable design parameters, features, modifications, advantages, and variations that are readily apparent to those skilled in the art in the field of occlusion devices.

### EXAMPLES

A study protocol with respect to the occlusion device disclosed herein and justification for animal use will be reviewed and approved by the Institutional Animal Care and Use Committee (IACUC) at ISIS Services and the procedures carried out under veterinarian supervision.

The rabbit elastase aneurysm model is a well-accepted and art-recognized model for testing novel neurointerventional devices and has been the subject of a number of clinical publications regarding efficacy and similarity to human response. (See, *e.g.,* Altes et al. Creation of Saccular Aneurysms in the Rabbit: A Model Suitable for Testing Endovascular Devices. AJR 2000; 174: 349-354.) It therefore is readily accepted by the regulatory agencies as an appropriate test model. The model's coagulation system is highly similar to that of humans. In addition, the model has advantageous anatomical aspects in that the diameters of the rabbits' extra-cranial carotid arteries are highly similar to the diameter of extra-cranial carotid arteries in humans. Moreover, elastase-induced aneurysms have been shown to behave in a histologically similar manner as human aneurysms.

A number of embodiments of the invention have been described. Without departing from the scope of the occlusion device disclosed herein, reasonable features, modifications, advantages, and design variations of the claimed apparatus will become readily apparent to those skilled in the art by following the guidelines set forth in the preceding detailed description and embodiments. Accordingly, other embodiments are within the scope of the following claims.

## Claims

1. An occlusion device for implantation into a body lumen or aneurysm (90), comprising:
one continuous mesh structure comprising axial mesh carriages (20) configured end to end, wherein the one continuous mesh structure is compressible; and
markers (50) encircling the one continuous mesh structure at pinch points (10) in the one continuous mesh structure,
wherein each end of each axial mesh carriage is (i) at a respective pinch point of the pinch points in the one continuous mesh structure and (ii) encircled by a respective marker of the markers, wherein each pinch point is a constrained and gathered location on the one continuous mesh structure,
wherein (i) the number of axial mesh carriages is equal to (n) and (ii) each axial mesh carriage is a respective segment of the one continuous mesh structure that has a width (y) and length (x) that is different from the length and width of adjacent carriages of the axial mesh carriages, and
wherein, for each axial mesh carriage, the width (y) is equal to or less than the length (x) in free air, and the width (y) is greater than the length (x) when compressed.

2. The occlusion device of claim 1, wherein each marker consists of wire strands wound around and therefore encircling a given pinch point.

3. The occlusion device of any of claims 1 to 2, wherein, when compressed, the axial length (x) (30) of each axial mesh carriage is about 5% to about 50% of its axial length in free air.

4. The occlusion device of any one of claims 1 to 3, wherein (n) is selected from the group consisting of two axial mesh carriages, three axial mesh carriages, four axial mesh carriages, and five mesh axial carriages.

5. The occlusion device of any one of claims 1 to 4, wherein the markers are radiopaque.

6. The occlusion device of any one of claims 1 to 4, wherein the continuous mesh structure comprises a metal braid comprising nitinol or a nitinol alloy.

7. The occlusion device of any one of claims 1 or 3 to 6, wherein each marker is a solid ring.

8. The occlusion device of any one of claims 1 or 3 to 6, wherein each marker is a solid band.

9. The occlusion device of claim 7 or 8, wherein each marker is formed from a least one material selected from the group consisting of: gold, platinum and stainless steel.

10. The occlusion device of any one of claims 1 to 9, wherein a width (y) of each axial mesh carriage in free air is between about 2 millimeters and about 50 millimeters.

11. The occlusion device of any one of claims 1 to 10, wherein, for each axial mesh carriage, the width (y) expands by a factor of two when the axial mesh carriage is compressed compared to when the axial mesh carriage is in free air.

12. A system for delivering an occlusion device to a body lumen or an aneurysm (90), comprising:
a. the occlusion device according to any one of claims 1 to 11; and
b. a delivery system configured to deliver the occlusion device to the body lumen or aneurysm.

## Patentansprüche

1. Okklusionsvorrichtung zur Implantation in ein Körperlumen oder Aneurysma (90), umfassend:
eine durchgehende Maschenstruktur umfassend axiale Maschenträger (20), die Ende an Ende angeordnet sind, wobei die eine durchgehende Maschenstruktur komprimierbar ist; und
Marker (50), die die eine durchgehende Maschenstruktur an Klemmstellen (10) in der einen durchgehenden Maschenstruktur umgeben,
wobei jedes Ende jedes axialen Maschenträgers (i) an jeweils einer Klemmstelle der Klemmstellen in der einen durchgehenden Maschenstruktur ist und (ii) von jeweils einem Marker der Marker umgeben ist, wobei jede Klemmstelle eine eingeengte und geraffte Stelle an der einen durchgehenden Maschenstruktur ist,
wobei (i) die Anzahl der axialen Maschenträger gleich (n) ist und (ii) jeder axiale Maschenträger ein jeweiliges Segment der einen durchgehenden Maschenstruktur ist, das eine Breite (y) und Länge (x) aufweist, die sich von der Länge und Breite benachbarter Träger der axialen Maschenträger unterscheiden, und
wobei für jeden axialen Maschenträger die Breite (y) im freien Zustand gleich oder kleiner als die Länge (x) ist und die Breite (y) im komprimierten Zustand größer als die Länge (x) ist.

2. Okklusionsvorrichtung nach Anspruch 1, wobei jeder Marker aus Drahtlitzen besteht, die um eine bestimmte Klemmstelle gewickelt sind und diese somit umschließen.

3. Okklusionsvorrichtung nach einem der Ansprüche 1 bis 2, wobei im komprimierten Zustand die axiale Länge (x) (30) jedes axialen Maschenträgers etwa 5 % bis etwa 50 % seiner axialen Länge im freien Zustand ist.

4. Okklusionsvorrichtung nach einem der Ansprüche 1 bis 3, wobei (n) aus der Gruppe ausgewählt ist, die aus zwei axialen Maschenträgern, drei axialen Maschenträgern, vier axialen Maschenträgern und fünf axialen Maschenträgern besteht.

5. Okklusionsvorrichtung nach einem der Ansprüche 1 bis 4, wobei die Marker röntgendicht sind.

6. Okklusionsvorrichtung nach einem der Ansprüche 1 bis 4, wobei die durchgehende Maschenstruktur ein Metallgeflecht umfasst, das Nitinol oder eine Nitinol-Legierung umfasst.

7. Okklusionsvorrichtung nach einem der Ansprüche 1 oder 3 bis 6, wobei jeder Marker ein massiver Ring ist.

8. Okklusionsvorrichtung nach einem der Ansprüche 1 oder 3 bis 6, wobei jeder Marker ein massives Band ist.

9. Okklusionsvorrichtung nach Anspruch 7 oder 8, wobei jeder Marker aus mindestens einem Material gebildet ist, das aus der Gruppe ausgewählt ist, bestehend aus: Gold, Platin und Edelstahl.

10. Okklusionsvorrichtung nach einem der Ansprüche 1 bis 9, wobei eine Breite (y) jedes axialen Maschenträgers im freien Zustand zwischen etwa 2 Millimetern und etwa 50 Millimetern liegt.

11. Okklusionsvorrichtung nach einem der Ansprüche 1 bis 10, wobei sich die Breite (y) jedes axialen Maschenträgers um den Faktor zwei vergrößert, wenn der axiale Maschenträgers zusammengedrückt wird, verglichen mit dem axialen Maschenträgers im freien Zustand.

12. System zum Einbringen einer Okklusionsvorrichtung in ein Körperlumen oder ein Aneurysma (90), umfassend:
a. die Okklusionsvorrichtung nach einem der Ansprüche 1 bis 11; und
b. ein Einbringungssystem, dazu ausgelegt, die Okklusionsvorrichtung in das Körperlumen oder das Aneurysma einzubringen.

## Revendications

1. Dispositif d'occlusion destiné à être implanté dans une lumière corporelle ou un anévrisme (90), comprenant:
une structure maillée continue comprenant des supports de mailles axiales (20) disposés bout à bout, ladite structure maillée continue étant compressible; et
des repères (50) entourant ladite structure maillée continue au niveau des points de pincement (10) de ladite structure maillée continue,
dans lequel chaque extrémité de chaque support de mailles axiales se trouve (i) au niveau d'un point de pincement respectif parmi les points de pincement de ladite structure maillée continue et (ii) est entourée par un marqueur respectif parmi les marqueurs, chaque point de pincement étant un emplacement contraint et froncé sur ladite structure maillée continue,
dans lequel (i) le nombre de supports de mailles axiales est égal à (n) et (ii) chaque support de mailles axiales constitue un segment respectif de ladite structure maillée continue, dont la largeur (y) et la longueur (x) diffèrent de la longueur et de la largeur des supports adjacents parmi les supports de mailles axiales, et
dans lequel, pour chaque support de mailles axiales, la largeur (y) est inférieure ou égale à la longueur (x) à l'air libre, et la largeur (y) est supérieure à la longueur (x) lorsqu'il est comprimé.

2. Dispositif d'occlusion selon la revendication 1, dans lequel chaque marqueur est constitué de brins de fil métallique enroulés autour d'un point de pincement donné et encerclant ainsi ce dernier.

3. Dispositif d'occlusion selon l'une quelconque des revendications 1 à 2, dans lequel, lorsque le support est comprimé, la longueur axiale (x) (30) de chaque support de mailles axiales représente environ 5 % à environ 50 % de sa longueur axiale à l'air libre.

4. Dispositif d'occlusion selon l'une quelconque des revendications 1 à 3, dans lequel (n) est choisi parmi le groupe constitué de deux supports de mailles axiales, de trois supports de mailles axiales, de quatre supports de mailles axiales et de cinq supports de mailles axiales.

5. Dispositif d'occlusion selon l'une quelconque des revendications 1 à 4, dans lequel les marqueurs sont radio-opaques.

6. Dispositif d'occlusion selon l'une quelconque des revendications 1 à 4, dans lequel la structure maillée continue est constituée d'une tresse métallique composée de nitinol ou d'un alliage de nitinol.

7. Dispositif d'occlusion selon l'une quelconque des revendications 1 ou 3 à 6, dans lequel chaque marquage est un anneau plein.

8. Dispositif d'occlusion selon l'une quelconque des revendications 1 ou 3 à 6, dans lequel chaque marqueur est une bande pleine.

9. Dispositif d'occlusion selon la revendication 7 ou 8, dans lequel chaque marqueur est constitué d'au moins un matériau choisi parmi le groupe comprenant : l'or, le platine et l'acier inoxydable.

10. Dispositif d'occlusion selon l'une quelconque des revendications 1 à 9, dans lequel la largeur (y) de chaque support de mailles axiales à l'air libre est comprise entre environ 2 millimètres et environ 50 millimètres.

11. Dispositif d'occlusion selon l'une quelconque des revendications 1 à 10, dans lequel, pour chaque support de mailles axiales, la largeur (y) double lorsque le support de mailles axiales est comprimé par rapport à lorsqu'il se trouve à l'air libre.

12. Système destiné à mettre en place un dispositif d'occlusion dans une lumière corporelle ou un anévrisme (90), comprenant:
a. le dispositif d'occlusion selon l'une quelconque des revendications 1 à 11; et
b. un système de mise en place conçu pour mettre en place le dispositif d'occlusion dans la lumière corporelle ou l'anévrisme.
